# EUROPEAN PATENT SPECIFICATION

(11) **EP 3 877 507 B1**
(45) Date of publication and mention of the grant of the patent: **11.02.2026**
(21) Application number: 19894370.6
(22) Date of filing: 27.11.2019
(51) Int. Cl.: C12Q 1/689

(54) **MINIMAL BIOMARKERS AND RELATED NEXT-GENERATION SEQUENCING KIT FOR DIAGNOSING ALZHEIMER'S DISEASE FROM INTESTINAL MICROBIOME**
MINIMALE BIOMARKER UND ZUGEHÖRIGES SEQUENZIERUNGSKIT DER NÄCHSTEN GENERATION ZUR DIAGNOSE VON MORBUS ALZHEIMER AUS EINEM INTESTINALEN MIKROBIOM
BIOMARQUEURS MINIMAUX ET KIT DE SÉQUENÇAGE DE NOUVELLE GÉNÉRATION ASSOCIÉ POUR DIAGNOSTIQUER LA MALADIE D'ALZHEIMER À PARTIR DU MICROBIOME INTESTINAL

(30) Priority: 03.12.2018 TR 201818373
(43) Date of publication of application: 15.09.2021
(73) Proprietor: T.C. Erciyes Universitesi, 38039 Kayseri (TR)
(72) Inventor: NALBANTOGLU, Özkan Ufuk, Kayseri (TR); GÜNDOGDU, Aycan, Talas/Kayseri (TR); KÖSEOGLU, Emel, Melikgazi/Kayseri (TR)
(74) Representative: Yalçiner Patent and Consulting Ltd.
(86) International application number: PCT/TR2019/051001
(87) International publication number: WO 2020/117164

(56) References cited:
- CN-A- 105 603 066
- US-A1- 2017 327 863
- US-A1- 2017 327 863
- US-B2- 9 909 191
- VOGT NICHOLAS M ET AL: "Gut microbiome alterations in Alzheimer's disease", SCIENTIFIC REPORTS,, vol. 7, 19 October 2017 (2017-10-19), pages 1 - 11, XP002788023, DOI: 10.1038/S41598-017-13601-Y
- VOGT NICHOLAS M ET AL: "GUT MICROBIOME ALTERATIONS IN ALZHEIMER'S DISEASE AND THE RELATIONSHIP WITH CSF BIOMARKERS", ALZHEIMER'S & DEMENTIA: THE JOURNAL OF THE ALZHEIMER'SASSOCIATION, vol. 13, no. 7, 1 July 2017 (2017-07-01), XP085218436, ISSN: 1552-5260, DOI: 10.1016/J.JALZ.2017.07.172
- ZHUANG ZHEN-QIAN ET AL: "Gut Microbiota is Altered in Patients with Alzheimer's Disease.", JOURNAL OF ALZHEIMER'S DISEASE : JAD 2018, vol. 63, no. 4, 30 May 2018 (2018-05-30), pages 1337 - 1346, XP009532899, ISSN: 1875-8908
- SHEN LIANG ET AL: "Alzheimer's Disease Histological and Behavioral Manifestations in Transgenic Mice Correlate with Specific Gut Microbiome State", JOURNAL OF ALZHEIMER`S DISEASE, IOS PRESS, NL, vol. 56, no. 1, 12 January 2017 (2017-01-12), pages 385 - 390, XP009520261, ISSN: 1387-2877, DOI: 10.3233/JAD-160884
- NALBANTOGLU, O. U.; SEZGIN, B.; GÜNDOGDU, A.; KOÇ, F.; GÖL, M. F. ; HANOĞLU, L.; KÖSEOGLU, E.; YILDIRIM S.: "Comparison of Gut Microbiota in Patients of Mild- Cognitive Impairment and Alzheimer's Diseasewith Age and Sex Matched Normal Controls: A Multicenter Study", THE 7TH ANNUAL INTERNATIONAL HUMAN MICROBIOME CONSORTIUM MEETING (IHMC2018) , KILLARNEY, IRELAND, 26 - 28 JUNE 2018, 28 June 2018 (2018-06-28), Killarney, Irland a, pages 165, XP009529005

## Description

### Technical Field of the Invention

The invention is related to a DNA sequencing kit and method.

### Known State of the Art (Prior Art)

Although the accurate diagnosis of Alzheimer's disease is conducted by determining amyloid plaque deposition in the brain by means of post-mortem autopsy, as this approach is irrelative in preventing and treating the disease, the present diagnosis methods, depend on mental status tests, physical examinations, neurologic screening and diagnosis with invasive bioindicators. Additionally, obtaining a protein panel from serum, a miRNA panel, genetic variations of APOEε4 gene, bioindicators depending on Aβ1-42 protein levels are still at the stage of being investigated and a known commercial product which is aimed to solve this diagnosis problem is not available.

US20160224749A1 (US15294526, US15679954, US15679903) and DK3171174T3 documents can be given as examples as being related to diagnosing Alzheimer's disease from the intestinal microbiome.

In the patent application numbered US20160224749A1 (US15294526, US15679954, US15679903) a microbial determination method depending on diagnosing diseases associated with microbiome taxonomic features in a subject has been disclosed. However, both said taxonomic groups do not match our suggested groups or genes and also a specific method in diagnosing Alzheimer's disease is not disclosed in this patent application.

In the registered patent numbered DK3171174T3, a method is disclosed which suggests prognosis of the disease according to the level of lactoferrin from saliva, in order to diagnose Alzheimer's disease. In this method, the lactoferrin agent that is to be measured is not related to the genetic determination materials suggested by us, and therefore our method is not related in any way with this registered patent.

In the study of "Gut microbiome alterations in Alzheimer's disease" (SciRep. 2017 Oct 19;7(1):13537. doi: 10.1038/s41598-017-13601-y), intestinal microbiome types that may be related to Alzheimer's disease has been investigated. In this study, although The biomarkers directed to diagnose Alzheimer's disease are not set forth directly, the taxonomic groups that are available in our suggestion have not been reported as significant groups carrying disease signals. Additionally, the study which guides our suggestion, is based on a Next-generation metagenomic sequencing technique where all bacterial genes are examined and the bioindicators are selected from sequenced genes; however in the above-mentioned article, any kind of bacterial gene has not been set forth which might be a biomarker and only 16S rRNA sequencing is carried out in order to reveal taxonomic group variations.

Alzheimer's disease diagnosis procedures based on the present clinical table or neurologic screening are not reliable standards and they are not very successful in terms of providing diagnosis accuracy. Due to this reason diagnosis approaches depending on molecular techniques are required.

As an alternative method samples are taken from the Cerebrospinal Fluid (CSF) for diagnosis and a decision is given by looking at the protein (most commonly Aβ1-42 protein) levels which are believed to be related to the disease. This operation causes discomfort for the patient (as it is an invasive operation) and medical risks depending on the operation are also present. However, if the patient does not accept to have the operation, the patient will not have a reliable option for the disease to be diagnosed. On the other hand, at the present time, the accuracy levels of even these tests have not been able to exceed the 80%-90% band.

At present, a cheap and reliable method or a kit based on genetic diagnosis which enables the diagnosis of the disease using non-invasive methods such as body fluids/secretions is not available.

A diagnosis method or a kit to be used specifically for Alzheimer's disease has not been disclosed in the patent application numbered US20160224749A1 (US15294526, US15679954, US15679903). On the other hand, our invention sets forth a new generation sequencing gene panel and a related kit formed of three different genes from the Lachnospiraceae family, one gene from the Eubacteriaceae family and one gene from the Bacteriodes vulgatus species. In the patent application mentioned, these taxonomic groups are not suggested for diagnosis, and they are not included In form of a gene panel or a kit..

In the registered patent, numbered DK3171174T3, diagnosing the disease from lactoferrin levels in saliva is suggested. The mentioned patent is related to diagnosing the disease by taking a saliva sample and measuring lactoferrin in the sample.

US2017327863 discloses a method and system for microbiome derived diagnostics and therapeutics. In this method a mental health associated condition in a subject is characterizing, diagnosing, and treating, involves determining a microorganism nucleic acid sequence and an alignment of the microorganism nucleic acid sequence to a reference nucleic acid sequence, generating at least one of a microbiome composition diversity dataset, and a microbiome functional diversity dataset based on the alignments, generating a characterization of the condition, determining a therapy for the subject, and providing therapy.

In the study of "Gut microbiome alterations in Alzheimer's disease" (SciRep. 2017 Oct 19;7(1):13537. doi: 10.1038/s41598-017-13601-y), the intestinal microbiome is examined in patients with Alzheimer's disease and healthy subjects as a control group and taxonomic groups that are believed to be related to the disease have been determined. The taxonomic groups comprising biomarker genes in our application do not match the taxonomic groups that have been set forth in the above-mentioned study. On the other hand, as the 16S rRNA sequencing used as a microbiome profiling technique in the related study cannot reveal microbial genes, Therefore the biomarkers at the gene level could not be set forth.

The method disclosed in the study of "Alzheimer's Disease Histological and Behavioral Manifestations in Transgenic Mice Correlate with Specific Gut Microbiome State" (J Alzheimers Dis. 2017;56(1):385-390. DOI: 10.3233/JAD-160884) aims to compare the gut microbiota in APP/PS1 transgenic mice of AD and C57/Bl6 wild-type (WT) mice by pyrosequencing the V3 and V4 regions of the bacterial 16S ribosomal RNA genes.

In the study of "Gut Microbiome Alterations in Alzheimer's Disease and The Relationship with CSF Biomarkers" (Alzheimer's & Dementia. 13. P563. DOI: 10.1016/j.jalz.2017.07.172.) relation between Alzheimer's disease and gut microbiota is investigated by examining the cerebrospinal fluid (CSF) biomarkers of AD were associated with the composition of the gut microbiome.

The study of "Gut Microbiome is Altered in Patients with Alzheimer's Disease" (Journal of Alzheimer's disease: JAD 63(4): 1-10, DOI:10.3233/JAD-180176) suggested that gut microbiota is altered in AD patients and may be involved in the pathogenesis of AD.

The study of "Comparison of Gut Microbiota in Patients of Mild-Cognitive Impairment and Alzheimer's Diseasewith Age and Sex Matched Normal Controls: A Multicenter Study.," (The 7th annual International Human Microbiome Consortium Meeting (IHMC2018) , Killarney, Ireland, pp.165, 2018) describes that Enterobacteriaceae was found to be enriched in AD patients and positively correlates with clinical scores. Differential abundance analysis revealed that Escherichia Shigella and Parabacteroides were enriched in AD; and Lachnospria and Roseburia in MCl.

### Brief Description of the Invention and its Aims

The invention is set out in the appended set of claims.

### Figures

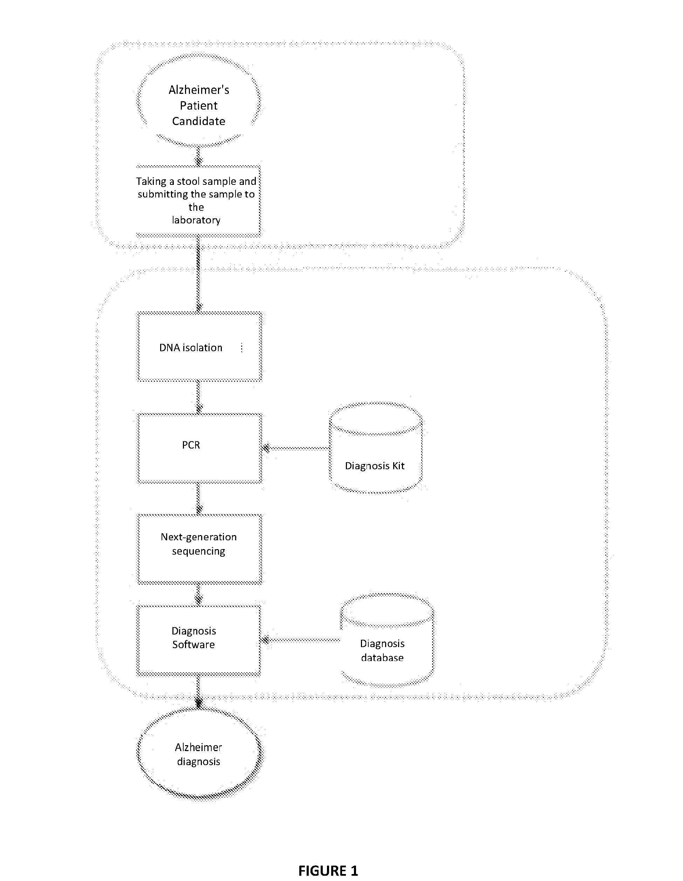

**Figure 1****:** Flow diagram of the method described in this invention.

### Detailed Description of the Invention

The developed method determines the DNA sequences of certain bacteria types that are associated with Alzheimer's disease and detects the disease signals of these bacteria from their relative amounts. However, the gene samples taken from the different genome regions of bacteria provide information regarding the life phase (e.g. increasing-decreasing populations) of the bacteria and these signals allow the diagnosis of the disease to reach a higher accuracy level. By means of the superiority provided by this technical difference, it is enabled for the invention to be more precise and accurate in comparison to other systems that diagnose Alzheimer's by means of bacteria levels. In the tests that have been carried out by using the kit and method of the invention, it has been determined that diagnosis can be performed with an accuracy level of 93%.

DNA sequences belonging to 3 different taxonomic groups that are located in human intestines and which are determined to be related to Alzheimer's disease have been obtained Using data mining techniques carried out on the intestinal metagenome of Alzheimer's disease patients and healthy subjects as a control group. 5 DNA sequences of similar size have been discovered as biomarkers. IT has been observed that as a result of the simulation and validation studies carried out in order to understand the success rate of the diagnosis of the disease using only these DNA sequences, was successful. Accordingly, the invention is formed of a DNA sequencing method, a DNA sequencing kit prepared to carry out this method.

By means of the invention evaluating the DNA obtained from the stool samples, have been produced.. On the other hand, as the method is based on next-generation DNA sequencing, due to the usage of this approach which is becoming more and more prevalent and whose cost is exponentially decreasing in time, this allows the method to be cheap, generic and to be provided as a solution that each small-scaled laboratory can access.

The DNA sequencing method respectively comprises the below-mentioned steps:
1) Taking a stool sample from the subject to be tested,
2) Reproducing the targeted DNA regions by means of Polymerase Chain Reaction (PCR) using 5 DNA primary pairs from the isolated DNA. 5 primer pairs have been provided below:
   1) F5'TAGCAGGTGATGATGCCAAC
      R5'AAATTTGAATGGGCTTGCTG
   2) F5'TGTATTTGGGCGAATTGTCA
      R5'AACGGTCTGCGATATGAACC
   3) F5'GCAGGCAGTGATCATTTTGA
      R5'AAGCGGATTACATCGGGATT
   4) F5'GGGGCTTAAGGTCAACATGA
      R5'AACGAATTCTTGCGTCCATC
   5) F5'TTGACTTGAACGACCTGCTG
      R5'GCAATCCCCGTATTCACACT
3) Sequencing the regions reproduced by PCR by means of next-generation DNA sequencing techniques.

The contents of the intestinal microbiome that is sequenced by means of the invention form minimal and optimal information related to Alzheimer's disease. Through the data mining studies that are carried out accordingly the least number of genetic materials have been determined. By means of the invention sequencing of low numbers of genetic targets, being able to conduct more tests with less unit of consumables, less labour and time, and therefore minimizing test costs and optimization of feasibility is provided. By this means both a simple and cheap sequencing kit is obtained.

### Procedure to be followed in order to determine primary pairs

First of all 30 Alzheimer's patients and 30 healthy control cohorts were determined. The patient group has been determined by diagnosis according to the Alzheimer's disease diagnosis criteria of National Institutes of Neurological and Stroke - Alzheimer's Disease and Related Disorders Association: NINDS-ADRDA. The neuropsychiatric examination has been conducted which includes the mini-mental status test in order to determine if the healthy subjects had dementia or not. It has been checked if the subjects included in the study had gastrointestinal diseases that may affect the intestinal microbiome or not and that they did not use antibiotics.

Study inclusion criteria for volunteers diagnosed with the disease
1. Being at the age of 65 or over the age of 65
2. Clinically having Alzheimer's type dementia according to NINDS-ADRDA criteria
3. Not having any gastrointestinal diseases that affect the intestinal microbiome and not using antibiotics
4. Not having a history of severe medicine or drug usage
5. Having determined B12 vitamin levels and not taking B12 vitamin supplements

Study inclusion criteria for healthy volunteers
1. Being at the age of 65 or over the age of 65
2. Not having dementia
3. Not having any psychiatric, neurologic and gastrointestinal diseases that affect intestinal microbiome
4. Not having any gastrointestinal diseases that affect the intestinal microbiome and not using antibiotics
5. Not having a history of severe medicine or drug usage
6. Having normal B12 vitamin levels, not taking B12 vitamin supplements

Following the selection of suitable patients and healthy volunteers, the stool samples of all of the participators (60 people) have been taken and placed into sterile sample cups in order to evaluate the intestinal microbiome of these subjects, the samples have been delivered to the laboratory in at most 4 hours with the condition that they are stored at +4 °C and total bacterial DNA isolation has been conducted using commercial kits. The amount and quality of the double-strand DNA (dsDNA) that has been isolated has been measured with a Qubit Fluorometer. DNA whose dsDNA quantification has been conducted with Qubit Fluorometer has been used for the entire genome sequencing. All of the metagenome sequencing studies have been carried out Using Illumina NextSeq 500 systems and Nextera enzymatic fractionation technology has been used during the preparation stage of preparing the samples for sequencing.

By means of whole metagenome sequencing it was aimed to detect microbial genes inside the intestinal microbiome of patients and healthy individuals, and then to set forth the duties and functional properties of these genes. It has been enabled for functional groups having statistically different relative abundance levels between control groups and subjects with the disease to be examined. In order for the genes to be able to be functionally examined, first of all, a determination needs to be carried out. Accordingly, first of all, the DNA readings that have been obtained by Illumina sequencing have been formed into longer fragments by genome assembly. IDBA_UD method has been used for genome Assembly, following, the oligonucleotide length Parameters determined as 80,90 and 100. Following this, the genes inside the assembled sequences have been determined by MetaGenMark and Prodigal programs as open reading frames. As genes appearing more than once should be identified as repeats and close homologs, in order to determine the real numbers of distinct genes these genes have been divided into groups by means of the CD-Hit method and during this division 97% similarity has been accepted as a grouping criteria for a gene. In order to characterize the metagenomes obtained with the next-generation sequencing system, at the level of gene ortholog groups, metabolic pathways, and system modules, first of all, examination was carried out by the BLASTP program, by taking the eggNOG database as reference, and then the relative abundances of these groups were mapped to the KEGG database and profiled at a metabolic pathway level.

As a result of this procedure, a total of approximately 10 million microbial genes and the relative abundances of these genes in each individual has been obtained. Therefore each individual has been represented with a profile having 10 million parameters. The most statistically valuable genes that can create a difference in subjects with the disease and subjects Without the disease within these profiles have been listed according to classification strengths via Gradient Boosted Trees algorithm and the least number of genes that shall increase the ROC AUC (area remaining below the ROC curve) value which evaluates classification strength have been determined by being added to the set of attributes using a greedy algorithm. It has been observed that during this selection, the first five genes that have been selected increased the ROC AUC value up to 0.9375 and have reached the largest value.

According to this, these five genes which were able to remain within the operational limits for multiplex polymerase chain reaction (mPCR) and which were able to reach the highest classification strength values have been selected. The amplicon regions directed to sequencing targeted DNA in order to determine relative abundance values of these genes and the required primary pairs for PCR amplification of these regions have been determined with Primer3 program.
5 primer pairs have been provided below:
1) F5'TAGCAGGTGATGATGCCAAC
   R5'AAATTTGAATGGGCTTGCTG
2) F5'TGTATTTGGGCGAATTGTCA
   R5'AACGGTCTGCGATATGAACC
3) F5'GCAGGCAGTGATCATTTTGA
   R5'AAGCGGATTACATCGGGATT
4) F5'GGGGCTTAAGGTCAACATGA
   R5'AACGAATTCTTGCGTCCATC
5) F5'TTGACTTGAACGACCTGCTG
   R5'GCAATCCCCGTATTCACACT

Criteria for determining these five primer pairs
i) the Next-generation sequencing technologies (e.g. Illumina MiSeq, NextSeq, HiSeq, NovaSeq; IonTorren, etc.) must match the DNA reading pair lengths, or in other words, they must be amplicon forming pairs at approximately 200-600 base-pair length band.
ii) they must have similar melting temperatures suitable for multiplex PCR reaction and
iii) they must not produce false-positive amplicons having a similar length on known genomes.

Accordingly
i) Primary pairs that are formed have been designed as:
   1. primer pair 284 base pair,
   2. primer pair 270 base pair,
   3. primer pair 269 base pair,
   4. primer pair 290 base pair, and
   5. primer pair 286 base pair
   and they meet the first criteria.
ii) All of the five primer pairs have been selected to have a melting temperature of a minimum of 57°C and a maximum of 63°C, therefore the second criteria were met.
iii) It was checked by the Primer Blast program at the NCBI database if all of the five primer pairs produced amplicons having a similar length at the genomes of the known type. It has been confirmed that each primer pair was at a distance of at least three nucleotides.

These pairs are are primer pairs that provide the above mentioned 3 primer selection criteria such that they shall represent the 5 biomarkers that have the highest classification performance according to the criteria described above. Due to this reason these pairs provide technical advantages due to mPCR and high disease classification strength in relation to other primer pairs that can be possibly selected.

The Lachnospiraceae family, Eubacterium genus and Bacteroidesvulgatus species, are bacteria species that carry the 5 genes that have been determined as biomarkers in the described procedure.

Accordingly the first two primer pairs target the gene regions that include "threonine synthesis" and "DUF4316 domain" respectively , the third primer pair targets the region of the gene of "intermembrane serine protease of the rhomboid family " located in Eubacterium genus, the fourth primer pair targets the gene region of "Group II intron reverse transcriptase" belonging to the Lachnospiraceae family and the fifth primer pair targets the region of the gene belonging to the "intermembrane serine protease of the rhomboid family" located in the Bacteroidesvulgatus species.

### An exemplary application of the DNA sequencing kit and method

The stool sample obtained from the individual to be tested is submitted to the laboratory and after the DNA isolation was carried out with commercial kits, 5 primary pairs of the kit of the Claim 1 are used to reproduce the gene regions determined with multiplex PCR. The obtained amplicon products are loaded to the Illumina MiSeq DNA sequencing system and samples belonging to at most 384 different patients are parallelly sequenced at the same time.

The reading files obtained by sequencing are analyzed with diagnosis software on a computer/workstation/smartphone and diagnosis results of the patients are obtained. This diagnostic part is not part of the invention and used for showing application where results of the DNA sequencing kit and method of Claim 1 can be used.

During the process mentioned above, the technologies used for DNA isolation, new generation sequencing and data analysis phases are tools/consumables that are already available in laboratory inventories in order to conduct several clinical tests. Therefore the invention is provided as a generic process based on the off-the-shelf principle which requires only PCR consumables and diagnosis software.

**Table 1: Table showing the success of the diagnosis method on the validation cohort by using the kit and method of the invention.**

| **ROC (area under the curve)** | Accuracy value | Precision value | Normalized Discounted Cumulative Gain |
|---|---|---|---|
| 0,9375 | 0,8333 | 0,9722 | 0,9866 |

As shown in Table 1, the disease diagnosis performance statistics according to the cross-validation tests carried out are presented as follows: The area remaining below the ROC characterization curve has been calculated as 0.9375. The average accuracy value of the correct positive and correct negative classifications has been measured as approximately 0.8333. The precision value which gives the correct diagnosis rate within all diagnoses is observed as 0.9722. The statistic of the normalized discounted cumulative gain which measures the correctness of the score grading of the diagnoses has been observed as 0.9866.

Using the Close relatives of the bacteria species used for diagnosing Alzheimer's, usage of the primers which enable to sequence the related regions by reproduction which is not included within the mentioned DNA primers or DNA regions belonging to the same genes or neighboring genes not included in the gene regions in the invention, can lead the way to be able to produce similar diagnostic products.

## Claims

1. DNA sequencing kit **characterized by** comprising the below mentioned 5 DNA primer pairs.
F5'TAGCAGGTGATGATGCCAAC
R5'AAATTTGAATGGGCTTGCTG
F5'TGTATTTGGGCGAATTGTCA
R5'AACGGTCTGCGATATGAACC
F5'GCAGGCAGTGATCATTTTGA
R5'AAGCGGATTACATCGGGATT
F5'GGGGCTTAAGGTCAACATGA
R5'AACGAATTCTTGCGTCCATC
F5'TTGACTTGAACGACCTGCTG
R5'GCAATCCCCGTATTCACACT

2. A DNA sequencing method, **characterized in that**
i. DNA isolation is conducted from a stool sample, and that it comprises the steps of,
ii. Reproducing the targeted DNA regions by means of Polymerase Chain Reaction (PCR) using the below listed 5 DNA primer pairs from isolated DNA,
F5'TAGCAGGTGATGATGCCAAC
R5'AAATTTGAATGGGCTTGCTG
F5'TGTATTTGGGCGAATTGTCA
R5'AACGGTCTGCGATATGAACC
F5'GCAGGCAGTGATCATTTTGA
R5'AAGCGGATTACATCGGGATT
F5'GGGGCTTAAGGTCAACATGA
R5'AACGAATTCTTGCGTCCATC
F5'TTGACTTGAACGACCTGCTG
R5'GCAATCCCCGTATTCACACT
iii. And sequencing the regions reproduced with PCR.

## Patentansprüche

1. DNA-Sequenzierungskit, **dadurch gekennzeichnet, dass** es die unten genannten 5 DNA-Primer paare umfasst.
F5' TAGCAGGTGATGATGCCAAC
R5'AAATTTGAATGGGCTTGCTG
F5' TGTATTTGGGCGAATTGTCA
R5'AACGGTCTGCGATATGAACC
F5' GCAGGCAGTGATCATTTTGA
R5'AAGCGGATTACATCGGGATT
F5' GGGGCTTAAGGTCAACATGA
R5'AACGAATTCTTGCGTCCATC
F5' TTGACTTGAACGACCTGCTG
R5' GCAATCCCCGTATTCACACT

2. DNA-Sequenzierungsverfahren, **dadurch gekennzeichnet, dass**
i. Die DNA-Isolierung wird aus einer Stuhlprobe durchgeführt und umfasst die folgenden Schritte,
ii. Die gezielten DNS-Regionen wurden aus isolierter DNA unter Verwendung der unten aufgeführten 5 DNA-Primer paare mittels Polymerase-Kettenreaktion (PCR) reproduziert,
F5' TAGCAGGTGATGATGCCAAC
R5' AAATTTGAATGGGCTTGCTG
F5' TGTATTTGGGCGAATTGTCA
R5' AACGGTCTGCGATATGAACC
F5' GCAGGCAGTGATCATTTTGA
R5' AAGCGGATTACATCGGGATT
F5' GGGGCTTAAGGTCAACATGA
R5' AACGAATTCTTGCGTCCATC
F5' TTGACTTGAACGACCTGCTG
R5' GCAATCCCCGTATTCACACT
iii. Und die Sequenzierung der mit PCR reproduzierten Regionen.

## Revendications

1. Kit de séquençage de l'ADN **caractérisé en ce qu'**il comprend les 5 paires d'amorces de l'ADN indiquées ci-dessous.
F5' TAGCAGGTGATGATGCCAAC
R5'AAATTTGAATGGGCTTGCTG
F5' TGTATTTGGGCGAATTGTCA
R5'AACGGTCTGCGATATGAACC
F5' GCAGGCAGTGATCATTTTGA
R5'AAGCGGATTACATCGGGATT
F5' GGGGCTTAAGGTCAACATGA
R5'AACGAATTCTTGCGTCCATC
F5' TTGACTTGAACGACCTGCTG
R5' GC AATCCCCGTATTCACACT

2. Procédé de séquençage de l'ADN, **caractérisé en ce que** :
i. Isoler l'ADN est réalisée à partir d'un échantillon de selles, et **en ce qu'**il comprend les étapes suivantes :
ii. Reproduire les régions de l'ADN ciblées au moyen d'une réaction en chaîne par polymérase (RCP) en utilisant les 5 paires d'amorces de l'ADN indiquées ci-dessous à partir de l'ADN isolé,
F5' TAGCAGGTGATGATGCCAAC
R5' AAATTTGAATGGGCTTGCTG
F5' TGTATTTGGGCGAATTGTCA
R5' AACGGTCTGCGATATGAACC
F5' GCAGGCAGTGATCATTTTGA
R5'AAGCGGATTACATCGGGATT
F5' GGGGCTTAAGGTCAACATGA
R5'AACGAATTCTTGCGTCCATC
F5' TTGACTTGAACGACCTGCTG
R5'GCAATCCCCGTATTCACACT
iii. Et séquencer les régions reproduites par PCR.
